# EUROPEAN PATENT APPLICATION

(11) **EP 4 159 869 A1**
(43) Date of publication of application: **05.04.2023**
(21) Application number: 21817696.4
(22) Date of filing: 31.05.2021
(51) Int. Cl.: C12P 19/04, C12N 1/20, C12R 1/01

(54) **PRODUCTION OF BACTERIAL NANOCELLULOSE**

(30) Priority: 01.06.2020 ES 202030505
(71) Applicant: Consejo Superior De Investigaciones Científicas (CSIC), 28006 Madrid (ES)
(72) Inventor: MARQUÉS MARTÍN, Silvia, 18008 GRANADA (ES); MARÍN QUERO, Patricia, 18008 GRANADA (ES); MARTIRANI VON ABERCRON, Sophie Marie, 18008 GRANADA (ES); PACHECO SÁNCHEZ, Daniel, 18008 GRANADA (ES)
(74) Representative: Pons
(86) International application number: PCT/ES2021/070387
(87) International publication number: WO 2021/245306

(57) **Abstract**

The present invention relates to a method for producing nanocellulose by means of culturing bacteria of the genus *Starkeya,* preferably from CO₂ as the sole carbon source, wherein, during their growth, the aforementioned bacteria synthesize a nanocellulose biofilm that they secrete into the extracellular medium. The present invention also describes a strain of *Starkeya* STN1A with an increased capacity for nanocellulose production in any culture medium.

## Description

The present invention relates to the use of bacteria of the genus *Starkeya* to produce nanocellulose and the use thereof in the manufacture of biofuels, in biomedicine, food and packaging. Therefore, the invention belongs to the field of biomaterials, more specifically to the technical field of the production of bacterial nanocellulose.

### BACKGROUND OF THE INVENTION

Cellulose is an organic biopolymer made up of glucose molecules, being one of the most abundant polymers and forming most of the terrestrial biomass. Cellulose and its possible derivatives can be used as raw material for a wide range of applications, such as biodiesel production, in food (e.g. hydrocolloid food additives), in cosmetics (e.g. gelling agents), in biomedicine (wound dressings, implants, catheters, drug release, etc.), or as part of materials for different applications (electronics, engineering, etc.), among others.

Currently, the world production of cellulose comes mainly from plants, wood and cotton being the primary sources of cellulose for most applications. This plant material has to undergo intensive processing for the removal of lignin, hemicellulose and other components from the plant matrix, with high economic and environmental costs, until it is purified. Both the chemical compounds used during the extraction process and the waste generated in the process involve environmental risks and serious waste disposal and/or recovery problems.

Furthermore, obtaining plant material requires extensive cultivation of best producer plant species (such as cotton), with the corresponding use of large agricultural areas, fertilizers, pesticides and fresh water for irrigation. Also, the use of hardwoods as a starting material requires the felling of trees, with the consequent problems of deforestation.

Bacterial cellulose or nanocellulose has been established as an important alternative to the use of plant cellulose. Cellulose produced from plant material requires intense chemical processing for purification, however, in the case of bacterial cellulose, the cellulosic material is recovered in a pure form with the removal of bacterial cells by means of a simple alkaline treatment.

Bacterial nanocellulose has become an emerging nanomaterial with physicochemical and mechanical properties that have expanded its use to different fields. Particularly its purity (since it lacks lignin and other materials derived from wood), high degree of polymerization and water retention capacity, as well as its good biocompatibility and low toxicity, make it an attractive material with promising prospects in biotechnology.

On the other hand, reducing the cost of producing the polymer could allow its use in more general applications, such as in the production of special papers, or specific packaging to maintain the humidity of fruits and vegetables for export after an appropriate modification of the fibers. For all these reasons, the production of cellulose by bacteria is considered a clean alternative for obtaining this polymer.

Although the synthesis of nanocellulose is a capability present in a good number of bacteria, the production of this polymer extracellularly forming long intertwined filaments, creating a mesh or biofilm, is an exclusive capability of some bacterial groups. Currently, the nanocellulose-producing bacterial strains described and used on the market are heterotrophic, requiring an organic carbon source for growth and for the production of the polymer.

There is a good number of bacteria the genome of which includes genes for the synthesis of nanocellulose. However, only a few groups can produce quality nanofibrillar cellulose in large quantities. Cellulose-producing bacteria *par excellence* are *Acetobacteraceae,* and among them especially *Gluconacetobacter xylinus* (renamed *Komagataeibacter xylinus),* and *Gluconacetobacter hansenii* are among the most efficient and best characterized producers *(*Charreau, H., et al. 2013, Recent Pat Nanotchenol 7, 56-80*).*

The basic production process consists of culturing nanocellulose-producing bacteria in the appropriate medium with various exogenously supplied organic carbon sources. The production of nanocellulose by these bacteria requires culture media rich in organic carbon sources, and the efforts for the optimization of culture media in order to reduce production costs and increase synthesis rates are continuous.

The production of cellulose by heterotrophic bacteria from organic substrates has been widely described and its optimization has been the subject of numerous studies and patents. For example, document US2018/0142274 A1 describes a strain of *Komagataeibacter* with enhanced nanocellulose production. Document CN101475973 describes a bacterial culture medium comprising a residual alcoholic liquor, water and sugar to obtain nanocellulose. Document JPH11181001 A describes the production of bacterial cellulose in a culture medium with polysaccharides such as carboxymethyl cellulose and aeration and stirring by, for example, *Acetobacter xylinum* subsp. *sucrofermentans.*

The state of the art has also described the production of bacterial nanocellulose from toxic organic compounds, such as by means of the cell culture of strains belonging to *Xanthobacteraceae,* capable of synthesizing nanocellulose from naphthalene crystals *(*Marin, P., Martirani-von Abercron, S.M., et al. 2019, Microb Biotechnol 12(4), 662-676*).*

Due to the need for an organic carbon source for the growth of nanocellulose-producing bacteria, excessive costs are required for the production of bacterial nanocellulose for common applications.

Furthermore, the excessive use of fuels in industrial activities and transport, have increased the concentrations of CO₂ emitted into the atmosphere in recent years, exacerbating the regenerative capability of the atmosphere to eliminate CO₂, which is the element primarily responsible for the greenhouse effect, affecting global climate change and global warming. Therefore, it is necessary to reduce both emissions and the CO₂ existing in the atmosphere.

For this reason, it is necessary to develop methods for the production of nanocellulose from preferably autotrophic microorganisms, that is, microorganisms that use inorganic carbon sources, such as, for example, CO₂, and excrete nanocellulose into the extracellular space during their growth in a virtually pure form without requiring complex methods for purification.

### DESCRIPTION OF THE INVENTION

The inventors have observed that bacteria of the genus *Starkeya* are capable of synthesizing a nanocellulose biofilm from carbon sources such as carbon dioxide (CO₂), glucose or glycerol. In that sense, the present description describes, for the first time, the production of bacterial nanocellulose through an autotrophic process, wherein the aforementioned bacteria extracellularly produce nanocellulose during their growth with CO₂. Furthermore, the nanocellulose thus obtained is pure nanocellulose, that is, after production there is no need to apply complex methods for its purification.

Obtaining the aforementioned nanocellulose biofilm was carried out by means of culturing bacterial strains of the genus *Starkeya,* specifically the strains *Starkeya* sp. STN1B (deposit number CECT 30087), *Starkeya* sp. STN1A (deposit number CECT 30088) and *Starkeya novella* DSM 506 (deposit number DSM 506), in the presence of glucose, crude glycerol or CO₂ as carbon sources, leading to the synthesis of extracellular nanocellulose.

Furthermore, the inventors observed that the strain *Starkeya* sp. STN1A was not only capable of producing a nanocellulose biofilm from CO₂, glucose, glycerol or naphthalene as carbon sources, but that in all cases the amount of nanocellulose produced by *Starkeya* sp. STN1A was significantly higher compared to the amount of nanocellulose produced by *Starkeya* sp. STN1B and *Starkeya novella* DSM 506, under the same conditions.

Using scanning electron microscopy, the inventors verified that the nanocellulose produced by bacteria of the genus *Starkeya* featured a three-dimensional lattice structure of nanofibers about 70 nm in diameter and several microns in length. This structure is characteristic of bacterial nanocellulose. The inventors also observed that the nanocellulose obtained consisted of 98.2% glucose.

Therefore, in a first aspect, the present invention relates to a method, hereinafter the "method of the invention", to produce nanocellulose comprising:
(a) culturing at least one microorganism belonging to the genus *Starkeya,* or a population comprising at least one microorganism belonging to the genus *Starkeya* in a culture medium comprising at least one carbon source, wherein the carbon source is not naphthalene, and
(b) purifying the nanocellulose biofilm obtained during step (a).

The term "nanocellulose" or "bacterial cellulose" or "nanofibrillar cellulose", used interchangeably herein, refers to a nanofibrillar polymer obtained by bacterial growth and made up of glucose units linked by bonds β (1→4), forming fibers between 10 and 100 nm in diameter and longer than one micron, capable of forming a three-dimensional lattice structure forming a cellulose biofilm.

The aforementioned nanocellulose biofilm is obtained during step (a) of the method of the invention by means of the microbiological growth or culturing of bacteria belonging to the genus *Starkeya.*

As understood by a person skilled in the art, the growth or culturing of a microorganism is carried out in a culture medium comprising the components necessary for the aforementioned microorganism to proliferate. In that sense, in the present document, the term "culture medium" refers to a substance, a compound, a mixture, a solution comprising the carbon source necessary for the growth and metabolism of the microorganism, wherein the aforementioned culture medium can be solid, semi-solid, semi-liquid or liquid.

In the present document, the term "carbon source" refers to molecules, chemical compounds, organic matter, which comprise carbon atoms (C) and are metabolized by microorganisms to carry out their growth and development, wherein the aforementioned carbon source can be of inorganic or organic origin.

Organic and inorganic carbon sources that can be used in the method of the invention are widely known in the state of the art. The main source of inorganic carbon in microbial growth is carbon dioxide (CO₂). In microbial growth, organic carbon sources or organic compounds are molecules which comprise carbon atoms forming covalent bonds with carbon and/or hydrogen, which can further have oxygen, nitrogen, sulfur, phosphor or halogens, among other elements, in their structure. Examples of organic compounds include, without being limited to, hydrocarbons, carbohydrates and lipids, which can be natural, synthetic or synthesized *in vivo* or *ex vivo* by living beings.

In the present document, the term "hydrocarbon" refers to molecules or chemical compounds formed by carbon (C) and hydrogen (H) atoms, which can have a linear, branched or cyclic structure and/or saturated bonds (for example, alkanes) or unsaturated bonds (for example, alkenes or alkynes) in their structure, and to compounds derived from hydrocarbons that may further comprise a functional group such as an ester, ether, aldehyde, ketone, carboxyl, hydroxyl, amine, amide, azo, nitro and sulfoxide group, among others. Examples of linear hydrocarbons include, without being limited to, methane, ethane, propane, butane, pentane, hexane, heptane, octane, nonane, decane, propene, propyne, butene and butyne. Examples of hydrocarbon derivatives include, without being limited to, glycerol, ethanol, methanol, isopropanol, propanol, formamide and methanoic acid. Examples of a cyclic or aromatic hydrocarbon (and derivatives thereof) include, without being limited to, benzene, toluene, anthracene and phenanthrene, among others.

In the present document, the term "carbohydrate" refers to molecules or chemical compounds formed by carbon (C), hydrogen (H), and oxygen (O) atoms. The main carbohydrates in the state of the art are glucides or sugars, formed by 3 or more carbon atoms in their structure, that can have two conformations forming two isomers, "D-isomer" or "L-isomer", and are classified according to their degree of polymerization as: monosaccharides, disaccharides, trisaccharides and polysaccharides.

Examples of monosaccharides, or a derivative obtained from a monosaccharide, include, without being limited to, glyceraldehyde, erythrose, threose, arabinose, arabitol, xylose, xylitol, lyxose, ribose, allose, altrose, glucose, mannose, mannitol, galactose, thallose, ribulose, fructose, sorbose, sorbitol, tagatose, gluconate and pyruvate, among others.

Examples of disaccharides include, without being limited to, saccharose, lactose, maltose, isomaltose, trehalose, nigerose and cellobiose, among others.

Examples of trisaccharides include, without being limited to, maltotriose, raffinose, nigerotriose, panose, melezitose, maltotriulose and kestose, among others. Examples of polysaccharides include, without being limited to, starch, glycogen, cellulose, chitin, alginate, dextran and alginic acid, among others.

In the present document, the term "lipid" refers to molecules or chemical compounds which comprise carbon and hydrogen and to a lesser extent oxygen, which can further comprise phosphorus, sulfur and nitrogen, and are hydrophobic. The main lipid carbon source are fatty acids. Examples of fatty acids include, without being limited to, phospholipid, glycolipids, triglycerides, propionic acid, butyric acid, palmitic acid, linoleic acid and oleic acid, among others.

Furthermore, the organic carbon source of the method of the invention can also be extracts of plant or animal materials, or byproducts in the synthesis or manufacture of materials or compounds such as, for example, polymers, fibres, plastics, detergents, soaps, biodiesel or dyes, among others. Examples of extracts or byproducts include, without being limited to, raw glycerin, yeast extract, soybean hydrolysate extract, fruit hydrolysate extract and molasses.

Any of these carbon sources can be used in the context of the present invention. Nevertheless, in a particular embodiment of the method of the invention, the carbon source comprises CO₂, glycerol or glucose, as well as any combination thereof.

As described in the examples herein, bacteria of the genus *Starkeya* are able to use the glycerol present in samples of crude glycerol or crude glycerin, thus allowing the use of residual byproducts for the synthesis of bacterial nanocellulose.

In another more particular embodiment, the carbon source is CO₂. The CO₂ can be provided directly in the form of air, preferably atmospheric CO₂, or in the form of sodium bicarbonate dissolved in the culture medium, or bubbling the medium with CO₂ by means of a gas bottle.

Furthermore, when the carbon source is CO₂, the culture medium has to comprise other components, such as a reducing compound that provides the energy and reducing power necessary for CO₂ fixation. The term "reducing compound" as used herein, refers to an electron-donating chemical compound or molecule that is oxidized by bacteria of the genus *Starkeya* in the method of the invention to obtain energy for carbon fixation and thus produce nanocellulose.

Therefore, in a particular embodiment, the culture medium used in the method of the invention further comprises a reducing compound. Any reducing compound can be used in the present invention. Examples of reducing compounds include, without being limited to, thiosulfate (S₂O₃²⁻), hydrogen sulfide (H₂S), inorganic sulfur (S₀), tetrathionate (S₄O₆²⁻), sulfite (SO₃²⁻), methanethiol (CH₃SH), dimethylsulfide (C₂H₆S, DMS) and dimethylsulfoniopropionate ((CH₃)₂S⁺CH₂CH₂COO⁻, DMSP). Nevertheless, in a particular embodiment of the method of the invention, the reducing compound is a reduced sulfur compound and/or hydrogen gas (H₂).

Likewise, the culture medium of the method of the invention may comprise supplementary compounds to aid bacterial growth. Examples of supplementary compounds include, but are not limited to, mineral salts, vitamins, trace elements, phosphate buffer, MOPS buffer (3-morpholino propane-1-sulfonic acid), Hepes buffer (2-[4-(2-hydroxyethyl)-1-piperazinyl]-ethanesulfonic acid), antibiotics, yeast extract, casamino acids, peptone, citric acid and EDTA (ethylenediaminetetraacetic acid) chelated iron.

As understood by a person skilled in the art, the culture medium used in the method of the invention to culture the bacteria of the genus *Starkeya* can be in solid, semi-solid, semi-liquid or liquid state. Nevertheless, in a particular embodiment, the culture medium used in the method of the invention is in a liquid state.

Liquid culture media for culturing bacteria of the genus *Starkeya* are known in the state of the art. Examples of media that can be used in the context of the present invention include, without being limited to, Widdel's modified mineral medium (WMM, Martirani von Abercron *et al.,* 2017), LB medium, PBT medium, mineral medium buffered at pH 7 with vitamins and/or trace elements.

Step (a) of the method of the invention comprises culturing a bacterium of the genus *Starkeya,* or a population comprising a bacterium belonging to said genus, in a culture medium comprising a carbon source, wherein said carbon source is not naphthalene.

The culture of the bacterium of the genus *Starkeya* has to be carried out under the right conditions so that the bacterium can grow and synthesize nanocellulose from the carbon source. Suitable pH, temperature, moisture conditions, etc., necessary to culture bacteria of the genus *Starkeya* are widely known in the state of the art. In general, the growth of bacteria of the genus *Starkeya* requires a temperature above about 10°C and below about 40°C. In this sense, in a particular embodiment of the method of the invention, step (a) is carried out at a temperature between 16°C and 40°C, preferably between 20°C and 36°C, more preferably between 24°C and 32°C. In another even more particular embodiment, step (a) of the method of the invention is carried out at a temperature of 28°C.

As understood by a person skilled in the art, the time that bacteria of the genus *Starkeya* have to be cultured to generate nanocellulose depends on the density of the inoculum, the production of nanocellulose required, the volume of the culture medium or the culture support surface. In another particular embodiment, step (a) of the method of the invention comprises a culture or incubation time of between 5 and 15 weeks.

The development and growth of bacteria belonging to the genus *Starkeya* not only depends on the carbon source, temperature or culture time, but also depends on other factors such as oxygen levels in the culture medium.

Oxygen levels in the culture medium during the growth of a bacterium of the genus *Starkeya* (step (a) of the method of the invention) can vary over a wide range, from low oxygen tensions or, in microaerophilic conditions, to high or saturating oxygen tensions, both with or without stirring.

Therefore, in another particular embodiment, step (a) of the method of the invention is carried out under microaerophilic conditions without stirring. The term "microaerophilic conditions" or "microaerophilic" as it is used in the present description, refers to the controlled intake of air to keep oxygen levels low, about 5% (particularly below 5 ppm) and at high carbon dioxide concentration between 5 and 10%.

In another particular embodiment, and alternatively to the previous embodiment, step (a) of the method of the invention is carried out under stirring conditions between 25 and 150 rpm without air intake control, preferably between 50 and 100 rpm, more preferably at 75 rpm.

Nevertheless, as understood by a person skilled in the art, if the method of the invention is to be optimized and the production of nanocellulose by bacteria of the genus *Starkeya* increased or accelerated, step (a) can be carried out under conditions of low oxygen concentrations in favor of high CO₂ concentrations because, as explained at the beginning of this description, the present invention is based on the discovery that bacteria of the genus *Starkeya* can produce nanocellulose from CO₂ as a carbon source.

Taxonomically, the genus *Starkeya* belongs to the family of *Xanthobacteraceae.* This genus currently includes the species *S. koreensis* and *S. novella.* Bacteria of the genus *Starkeya* are chemolithoautotrophic, facultative heterotrophic bacteria, that is, bacteria capable of growing in a culture medium using inorganic compounds and/or organic compounds as a carbon source.

In putting the method of the invention into practice, the inventors used the bacterial strains of the genus *Starkeya* referred to herein as *Starkeya* sp. STN1B and *Starkeya* sp. STN1A. Strains STN1B and STN1A comprise a genome with less than 95% mean nucleotide sequence identity compared to the genome of an *S. novella* type strain DSM 506.

This is indicative that strains STN1B and STN1A do not belong to the species *S. novella,* or to *S. koreensis,* and therefore would be a new species of the genus *Starkeya.* Therefore, any bacterium of the genus *Starkeya* can be used in the method of the invention, such as *Starkeya novella* DSM 506, *Starkeya* sp. STN1B (deposit number CECT 30087) or strain *Starkeya* sp. STN1A (deposit number CECT 30088).

Strain *Starkeya* sp. STN1B was isolated from an enrichment culture of a hydrocarbon-contaminated aquifer sample, cultured in Widdel's modified mineral medium (WMM) with naphthalene crystals as the sole carbon source, as described in Martirani von Abercron et al., 2017, Microb. Biotechnol. 10(6), 1781-1796. Strain *Starkeya* sp. STN1B was deposited on 25 February 2020 under the Budapest Treaty in the Spanish Type Culture Collection as the International Deposit Authority (main office at Edificio 3 CUE, Parc Científic Universitat de Valencia, C/ Catedrático Agustín Escardino, 9, 46980 Paterna (Valencia) SPAIN). The assigned deposit number was CECT 30087. The depositor of the strain was the Consejo Superior de Investigaciones Científicas (CSIC).

Strain *Starkeya* sp. STN1A was isolated due to having a different colony morphology from pure cultures of strain STN1B in WMM medium with naphthalene as the sole carbon source and dilution seedings in solid WMM medium with LB diluted 1/10 as the carbon source. Strain *Starkeya* sp. STN1A was deposited on 25 February 2020 under the Budapest Treaty in the Spanish Type Culture Collection as the International Deposit Authority (main office at Edificio 3 CUE, Parc Científic Universitat de Valencia, C/ Catedrático Agustín Escardino, 9, 46980 Paterna (Valencia) SPAIN). The assigned deposit number was CECT 30088. The depositor of the strain was the Consejo Superior de Investigaciones Científicas (CSIC).

The strain under deposit number CECT 30088 *(Starkeya* sp. STN1A) is a spontaneous mutant strain of the strain under deposit number CECT 30087 *(Starkeya* sp. STN1B) comprising a stable deletion of a 62-65 kb fragment of the genome, as well as a series of point mutations. This strain presents a significant increase in the capability for the synthesis of nanocellulose with any carbon source in the culture medium.

Nevertheless, in a particular embodiment, the bacterium of the genus *Starkeya* is the strain of *Starkeya* sp. STN1B (deposit number CECT 30087) or strain *Starkeya* sp. STN1A (deposit number CECT 30088).

Lastly, the method of the invention comprises a step b) comprising the purification of the nanocellulose biofilm from the extracellular medium. It is routine practice for one skilled in the art to determine methods of purifying nanocellulose from the extracellular medium. Examples of nanocellulose purification methods include, without being limited to, decantation, washings with bidistilled water, cell hydrolysis through alkaline treatments, filtration, precipitation, centrifugation, sedimentation, treatment with sodium hypochlorite, treatment with surfactant agents, treatment with potassium hydroxide, treatment with sodium hydroxide, extraction with organic solvents and heating.

By means of the method of the invention, nanocellulose polymers are produced from any carbon source, preferably from CO₂. Therefore, another aspect of the present invention is a nanocellulose polymer, hereinafter "polymer of the invention", obtained or obtainable by the method of the invention.

The term "nanocellulose" has been defined in previous paragraphs of this document and applies equally to this aspect of the invention.

Using scanning electron microscopy, the inventors verified that the polymer of the invention featured a three-dimensional lattice structure of nanofibers about 70 nm in diameter and several microns in length. The inventors also observed that the nanocellulose obtained consisted of 98.2% glucose.

As understood by a person skilled in the art, the polymer of the invention may be contained within a composition. In that sense, another aspect of the present invention relates to a composition comprising the polymer of the invention, hereinafter "first composition of the invention".

Due to the capability for the production of nanocellulose of bacteria belonging to the genus *Starkeya,* another aspect of the present invention is the use of a microorganism belonging to the genus *Starkeya,* or a population comprising said microorganism, hereinafter "use of the invention", for the production of nanocellulose, wherein the production of nanocellulose is carried out in a culture medium comprising at least one carbon source, wherein the carbon source is not naphthalene.

The terms "nanocellulose", "genus *Starkeya",* "culture medium" and "carbon source" have been defined in previous paragraphs of this document and apply equally to this aspect of the invention, as well as all its particular embodiments (alone or in combination).

In that sense, in another particular embodiment of the use of the invention, the carbon source is selected from the list consisting of CO₂, glycerol, glucose and any combination thereof.

In another more particular embodiment of the use of the invention, the carbon source comprises CO₂. The CO₂, can be provided directly in the form of air, preferably atmospheric CO₂, or in the form of sodium bicarbonate dissolved in the culture medium, or bubbling the medium with CO₂ by means of a gas bottle.

In addition to the carbon source, the culture medium for the use of the invention may comprise an electron-donating chemical compound or molecule to provide energy, referred to as a reducing compound. In that sense, in another particular embodiment of the use of the invention, the culture medium for the production of nanocellulose further comprises a reducing compound. Particularly, when the carbon source is CO₂, the culture medium must comprise at least one reducing compound that provides the energy and reducing power necessary for CO₂ fixation.

The term "reducing compound" has already been described in other previous aspects of the invention and applies equally to the present aspect. In another particular embodiment of the use of the invention, the reducing compound is a reduced sulfur compound and/or hydrogen gas (H₂). Examples of reduced sulfur compounds include, without being limited to, thiosulfate (S₂O₃²), hydrogen sulfide (H₂S), inorganic sulfur (S0), tetrathionate (S₄O₆²⁻), sulfite (SO₃²), methanethiol (CH₃SH), dimethylsulfide (C₂H₆S, DMS) and dimethylsulfoniopropionate ((CH₃)₂S⁺CH₂CH₂COO⁻, DMSP).

Furthermore, the culture medium used in the use of the invention may comprise supplementary chemical compounds to aid bacterial growth during the production of nanocellulose. Examples of supplemental chemical compounds include, without being limited to, mineral salts, vitamins, trace elements, buffering compounds such as phosphate buffer, MOPS buffer (3-morpholino propane-1-sulfonic acid), Hepes buffer (2-[4-(2-hydroxyethyl)-1-piperazinyl]-ethanesulfonic acid), antibiotics, yeast extract, casamino acids, peptone, citric acid and EDTA (ethylenediaminetetraacetic acid) chelated iron.

As in the method of the invention, any microorganism of the genus *Starkeya* can be used in the production of nanocellulose. Nevertheless, in a particular embodiment of the use of the invention, the microorganism of the genus *Starkeya* is selected from the list consisting of strain *Starkeya* sp. STN1B under deposit number CECT 30087 and strain *Starkeya* sp. STN1A under deposit number CECT 30088.

Strains STN1B and/or STN1A belonging to the genus *Starkeya* have already been described in other previous aspects of the invention and apply equally to this aspect of the invention.

The strain under deposit number CECT 30088 *(Starkeya* sp. STN1A) presents a significant increase in capability for the synthesis of nanocellulose, therefore, in another more particular embodiment of the use of the invention, the microorganism belonging to the genus *Starkeya* for the production of nanocellulose is *Starkeya* sp. STN1A.

As described hereinabove, the production of nanocellulose develops during the growth of bacteria of the genus *Starkeya,* which require a culture medium comprising at least one carbon source and a temperature above about 10 °C and below about 40 °C. In that sense, in a particular embodiment of the use of the invention, the growth/culturing of the bacterium of the genus *Starkeya* is carried out at a temperature between 16°C and 40°C, preferably between 20°C and 36°C, more preferably between 24°C and 32°C. In another even more particular embodiment of the use of the invention, growth/culturing is carried out at a temperature of 28°C.

In another particular embodiment of the use of the invention, the culture medium is liquid. Liquid culture media for culturing bacteria of the genus *Starkeya* have been previously described in the present description and are applicable to the use of the invention.

It is also routine practice for one skilled in the art to determine the culture time required for the bacterium of the genus *Starkeya,* or a bacterial population comprising said bacterium, to produce nanocellulose. As already explained, the aforementioned culture time depends on the density of the inoculum, the volume of the culture medium or the culture support surface. In another particular embodiment of the use of the invention, the production of nanocellulose is carried out in a culture or incubation time of between 5 and 15 weeks.

In the use of the invention, the production of nanocellulose not only depends on the carbon source and the culture medium, temperature or culture time, but also on oxygen levels during microbial growth.

In that sense, in another particular embodiment, the production of nanocellulose of the use of the invention is carried out under microaerophilic conditions and without stirring. The term "microaerophilic conditions" has been defined in previous paragraphs and is applicable to the present embodiment.

In another particular embodiment, and alternatively to the previous paragraph, the production of nanocellulose is carried out under stirring conditions between 25 and 150 rpm without air intake control, preferably between 50 and 100 rpm, more preferably at 75 rpm.

The culturing of strain *Starkeya* sp. STN1A isolated by the inventors gave rise to a significant increase in the capability for the synthesis of nanocellulose, regardless of the carbon source used for its growth. Therefore, strain *Starkeya* sp. STN1A presents improved properties in the production of nanocellulose compared to other bacteria of the genus *Starkeya.*

Accordingly, another aspect of the invention relates to a strain of the genus *Starkeya,* under deposit number CECT 30088 *(Starkeya* sp. STN1A), hereinafter "strain of the invention".

As used herein, the term "strain" refers to a genetic variant or subtype of an organism of the genus *Starkeya.*

Also contemplated in the present invention is a strain derived from the strain of the invention under deposit number CECT 30088 (STN1A).

As used herein, the terms "mutant" or "mutant strain" or "derived" or "derived strain" are equivalent and refer to any microorganism that results from the mutation or change in the DNA of one or more genes of the strain *Starkeya* sp. under deposit number CECT 30088 *(Starkeya* sp. STN1A) and that essentially maintains the properties of the synthesis of nanocellulose, as described in the present invention. The derived strain can be produced naturally or intentionally by mutagenesis methods known in the state of the art such as, by way of example, the growth of the original microorganism in the presence of mutagenic or stress-causing agents, or by genetic engineering aimed at modifying specific genes.

As is known to a person skilled in the art, the strain of the invention or the derived or mutant strain may be comprised in a bacterial population. Therefore, another aspect of the present invention relates to a bacterial population comprising the strain of the invention or the derived or mutant strain thereof, hereinafter referred to as "bacterial population of the invention".

The terms "strain", "derived or mutant strain", have been defined in previous paragraphs of this document and apply equally to this aspect of the invention.

As understood by a person skilled in the art, both the strain of the invention, the derived or mutant strain or population of the invention, may be contained within a composition.

In that sense, in another aspect, the present invention relates to a composition comprising the strain of the invention, the derived or mutant strain thereof, or the population of the invention, as well as any combination thereof, hereinafter "second composition of the invention", including all the particular embodiments defined in the preceding paragraphs.

Due to the enhanced capability of the strain under deposit number CECT 30088 *(Starkeya* sp. STN1A) for the synthesis of nanocellulose, regardless of the carbon source used for its growth, another aspect of the present invention relates to the use of the strain of the invention, or of the bacterial population of the invention, or the second composition of the invention, for the production of nanocellulose, hereinafter, the "second method of the invention".

The production of nanocellulose of the second use of the invention is carried out in a culture medium comprising at least one carbon source, wherein the carbon source can be of inorganic or organic origin. The terms "nanocellulose", "culture medium" and "carbon source" have already been described in previous aspects herein, and these, as well as their particular embodiments, apply equally to this inventive aspect.

In another particular embodiment, the carbon source of the second use of the invention is CO₂, naphthalene, glycerol or glucose, as well as any combination thereof, preferably the carbon source is CO₂.

In another particular embodiment, the culture medium of the second use of the invention further comprises a reducing compound. The term "reducing compound" has already been described in previous aspects herein, and these, as well as their particular embodiments, apply equally to this particular embodiment.

Microbial growth of the strain under deposit number CECT 30088 *(Starkeya* sp. STN1A) requires the same conditions of temperature, state of the culture medium, culture time, oxygen levels of the culture medium as in previously described inventive aspects, as well as their particular embodiments, and they apply equally to this inventive aspect.

Throughout the description and the claims, the word "comprises" and its variants do not intend to exclude other technical features, additives, components or steps. For those skilled in the art, other objects, advantages and features of the invention may be partially deduced from both the description and the embodiment of the invention. The following examples and figures are provided by way of illustration and are not intended to limit the present invention.

### BRIEF DESCRIPTION OF THE FIGURES

**Figure 1****.** Scanning electron microscopy images of the biofilm produced by a culture of strain STN1B growing in PBT medium. Left, view of the biofilm at 20.00 KX. Right, detail at 50.00 KX to estimate the diameter of the fibers, about 70 nm.
**Figure 2****.** Attenuated total reflectance Fourier transform infrared spectroscopy (ATR-FTIR) spectrum of a cellulose membrane produced by strain STN1B in culture with bicarbonate (CO₂) as the sole carbon source.
**Figure 3****. A and B)** Scanning electron microscopy images of biofilms produced by strain STN1A growing in PBT medium. A) view of the biofilm at 10.00 KX. B) detail at 30.00 KX. **C)** Scanning electron microscopy images of biofilms produced by strain STN1A growing with glucose as the carbon source (10.00 KX).
**Figure 4****.** ATR-FTIR spectrum of a cellulose membrane produced by strain STN1A in culture with bicarbonate (CO₂) (A), or glucose (B), as the sole carbon source.
**Figure** 5. Image of the nanocellulose synthesized by strains STN1B, STN1A and DSM 506 growing with glucose as the carbon source.
**Figure 6****.** Scanning electron microscopy image of a biofilm produced by strain S. *novella* DSM 506 in PBT culture medium with bicarbonate (CO₂) as the sole carbon source.

### EXAMPLES

Next, the invention will be illustrated by means of assays carried out by the inventors which demonstrate the effectiveness of the invention.

### MATERIALS AND METHODS

### Bacterial strains

Strain *Starkeya* sp. STN1B was deposited on 25 February 2020 under the Budapest Treaty in the Spanish Type Culture Collection as the International Deposit Authority (main office at Edificio 3 CUE, Parc Científic Universitat de Valencia, C/ Catedrático Agustín Escardino, 9, 46980 Paterna (Valencia) SPAIN). The assigned deposit number was CECT 30087. The depositor of the strain was the Consejo Superior de Investigaciones Científicas (CSIC).

Strain *Starkeya* sp. STN1A was deposited on 25 February 2020 under the Budapest Treaty in the Spanish Type Culture Collection as the International Deposit Authority (main office at Edificio 3 CUE, Parc Científic Universitat de Valencia, C/ Catedrático Agustín Escardino, 9, 46980 Paterna (Valencia) SPAIN). The assigned deposit number was CECT 30088. The depositor of the strain was the Consejo Superior de Investigaciones Científicas (CSIC).

Strain Starkeya novella DSM 506 *(*Kelly et al., 2000, Int J Syst Evol Microbiol 50, 1797-1802*),* isolated by R.L. Starkey in 1934, was deposited under the Budapest Treaty in the German Collection of Microorganisms and Cell Cultures (DSMZ) as the International Depository Authority, located at the Leibniz Institute DSMZ, Inhoffenstraße 7B, 38124, Braunschweig (Germany). The assigned deposit number was DSM 506.

### Isolation of bacterial strains

Strain *Starkeya* sp. STN1B was isolated from an enrichment culture of a hydrocarbon-contaminated aquifer sample, cultured in Widdel's modified mineral medium (WMM) with naphthalene crystals as the sole carbon source, as described in Martirani von Abercron et al., 2017, Microb. Biotechnol. 10(6), 1781-1796. The cultures were kept in 120 ml bottles filled with 75 ml of the medium and capped with a butyl stopper wrapped in a layer of Teflon tape to allow controlled airflow. The bottles were sealed with an aluminum closure with a central hole. After several enrichment passages (every 1 or 2 months, 10% (v/v) of the culture was transferred to fresh medium), the bacterial community was dominated by two strains, one of which was *Starkeya* sp. STN1B, which was isolated by the inventors on solid WMM medium (1.8% agar) with naphthalene as the sole carbon source.

WMM medium contains: 30 mM MOPS buffer (3-(N-morpholino)propanesulfonic acid) at pH 7.2; 8 mM KNOs; 17.1 mM NaCl; 6.7 mM KCI; 0.6 mM CaCl₂; 4.7 mM NH₄Cl; 2 mM MgCl₂; 1.44 mM KH₂PO₄; 1 mM Na₂SW₄. For every 50 ml, the solution is supplemented with 15 µl of a vitamin solution, 50 µl of the trace element SL10 solution, 50 µl of a selenium-tungsten solution and biotin to a concentration of 1 µM.

Strain *Starkeya* sp. STN1A was isolated from pure cultures of strain STN1B in WMM medium with naphthalene as the sole carbon source and in serial dilutions in solid WMM medium with LB diluted 1/10 as the carbon source, because of its different colony morphology from colonies of strain STN1B. Undiluted LB medium contained, in 1 liter of water, 10 g of bactotryptone, 5 g of yeast extract and 10 g of sodium chloride. With some frequency, the resulting colonies appeared with a different morphology (pale and transparent). The analysis of these colonies showed that it was a strain of *Starkeya* spp. that was missing a chromosome fragment and had a greater capability to synthesize cellulose, resulting in a new strain of the genus *Starkeya,* which has been deposited under deposit number CECT 30088.

Mutated strain *Starkeya* sp. STN1B *bcsA* was obtained by the inventors by means of deleting a part of the gene *bcsA,* the nucleotide sequence of which is SEQ ID NO: 1, corresponding to cellulose synthase, and therefore deficient in the synthesis of cellulose. The sequence of the gene cluster for the synthesis of cellulose in *Starkeya* sp. STN1B and STN1A, which includes the gene *bcsA,* is deposited in the GenBank database under accession number MK350252. The inventors constructed it by double homologous recombination as described in Marin et al., Microb. Biotechnol. 2019 12(4),662-676. The part of the deleted gene *bcsA* (SEQ ID NO: 1) synthesizes an inactive protein with a deletion spanning between residues Ser285 and Pro689. Strain *Starkeya* sp. under deposit number CECT 30088 (STN1A) mutated in the gene *bcsA* (SEQ ID NO: 1) for cellulose synthase, and therefore deficient in the synthesis of cellulose, was constructed by the inventors by double homologous recombination, following the same protocol used for the strain STN1B mutant.

### Culture media and conditions for the production of nanocellulose

The inventors grew the strains under microaerophilic conditions at 28 °C and without stirring, in 50 or 100 ml bottles, filled up to 80% of their capacity with the PBT medium, or in flasks of different volumes (between 100 and 500 ml) and filled up to 40% of their capacity with the PBT medium.

A 40 ml culture of PBT medium should contain: 50 mM phosphate buffer at pH 7.2; 8 mM KNO₃; 17.1 mM NaCl; 6.7 mM KCI; 0.6 mM CaCl₂; 4.7 mM NH₄Cl; 2 mM MgCl₂; 1.44 mM KH₂PO₄; 1 mM Na₂SW₄. For every 40 ml, the solution is supplemented with 12 µl of a vitamin solution, 40 µl of the trace element SL10 solution, 40 µl of a selenium-tungsten solution and biotin to a concentration of 1 µM. Phosphate buffer is obtained by diluting 5 times a prepared 0.5 M stock solution, mixing, in 1 liter of water, 21.8 g of NaH₂PO₄·2H₂O and 64.07 g of NaH₂PO₄·2H₂O, and adjusting the final pH to 7.2.

The vitamin solution is prepared in 10 mM phosphate buffer at pH 7.1 and contains in 100 ml: 4 mg of 4-aminobenzoic acid, 1 mg of D(+)-biotin, 10 mg of nicotinic acid, 5 mg of D(+) calcium pantothenate, 15 mg of pyridoxine dihydrochloride. The trace element solution is prepared by adding to one liter of water: 13 ml of 25% HCl; 10 mg of H₃BO₃; 5 mg of MnCl₂·4H₂O; 2.1 g of FeSO₄·7H₂O; 190 mg of CoCl₂·6H₂O; 24 mg of NiCl₂·6H₂O; 2 mg of CuCl₂·2H₂O; 144 mg of ZnSO₄·7H₂O. The selenium-tungsten solution is prepared by adding to 1 liter of water: 400 mg of NaOH, 6 mg of Na₂SeO₃·5H₂O, 36 mg of Na₂MoO₄·2H₂O, 8 mg of Na₂WO₄·2H₂O. Sodium thiosulfate is added from a 200 mM stock solution, obtained by dissolving 50 g of Na₂S₂O₃·5H₂O in 1 liter of water.

Lastly, the medium is supplemented, if applicable, with 20 mM sodium bicarbonate as the carbon source and 40 mM sodium thiosulfate (final concentrations). Optionally, the antibiotics kanamycin at 5 µg/ml and nalidixic acid at 10 µg/ml can be added in the medium.

The inventors inoculated the aforementioned culture media with strains DSM 506, STN1B or STN1A, described above, from a saturated culture, with the quantity necessary to obtain a culture with an initial turbidity, measured as absorbance A₆₆₀, between 0.01 and 0.005. Once the strains were inoculated, the inventors capped the culture bottles with a butyl stopper and sealed them with an aluminum closure with a central hole, allowing the plug to be pierced with a 30-gauge hypodermic needle filled with cotton fiber to allow the controlled passage of sterile air. In the case of cultures in flasks, these were covered with a hydrophilic cotton plug. This process was carried out with previously sterilized material and under sterile conditions.

For larger volumes, the inventors used containers of different glass and plastic materials, such as glass Petri dishes 18 cm in diameter or covered borosilicate or polypropylene trays 5-10 cm deep and with 160-1400 cm² of surface area, filled to half their volume with PBT medium. They inoculated the cultures in trays with the proportional amount of culture, which were kept static at 28°C.

When the carbon source is glucose or glycerol, the conditions and culture medium are the same, with the exception of bicarbonate and thiosulfate, which were removed from the medium, and the inventors added glycerol or glucose at a final concentration of 50 and 25 mM, respectively.

### Preparation of samples for the characterization of cellulose

Once the nanocellulose biofilm was synthesized in the culture medium, the inventors collected it by decantation, transferring it to glass flasks, where they washed it by incubation in bi-distilled water for 24-48 hours with frequent water changes. To eliminate bacterial cells, the inventors treated the biofilm with a 1% aqueous KOH solution with gentle stirring (50 rpm) for 24-48 hours.

Subsequently, they removed this solution and subjected the biofilm to continuous washings with bi-distilled water for 24-48 hours, changing the water several times until a neutral pH is obtained. After cell hydrolysis and washing, and prior to the characterization of the polymer, the aforementioned polymer was drained and frozen at -80°C for 24 hours for subsequent lyophilization, carried out with a VirTis model Benchtop freeze dryer at - 100 °C with a vacuum pressure of 7 mT for 5-12 hours.

For the determination of the dry weight of the nanocellulose produced by the three strains, once the bacteria were eliminated with KOH and washed with distilled water, wet samples were dried for several days at 50°C, controlling the decrease in the weight of the material until it remained stable after several measurements. These final values were taken as dry weight.

### Characterization of nanocellulose

The inventors confirmed the chemical composition of cellulose after its chemical hydrolysis by: i) deconstruction of cellulose by completely dissolving 10 mg of the lyophilized polymer in 190 mg of the ionic liquid 1-ethyl-3-methylimidazolium chloride [EMIM][CI] at 140°C for 1.5-2 hours of incubation, ii) subsequent reconstruction of the structure by precipitation with 1 ml of cold MilliQ water and washing of the precipitate with MilliQ water, and iii) hydrolysis of the resulting precipitate, resuspended in 0.8 ml of MilliQ water, by addition of sulfuric acid to reach a final 0.2 M, and incubation at 140°C for 5 hours.

The inventors filtered the resulting solution to quantify the non-hydrolyzed solid material and analyzed the resulting liquid by means of GC-MS at the Scientific Instrumentation Services of the Zaidín Experimental Station, CSIC, Granada. To that end, the samples were dried with nitrogen gas and derivatized with 150 µl of oximation reagent and later with 100 µl of silylation reagent (both reactions at 75°C for 30 minutes). In this way, the inventors obtained the trimethylsilyloxime derivatives of the sugars present, which they analyzed in a Varian 450GC 240MS equipment injecting 1 µl at 230°C in splitless mode, with an He flow at 1 ml/min and a temperature ramp from 150°C for 5 min at 5°C/min up to 250°C, followed by a ramp at 30°C/min up to 310°C for 3 min.

Subsequently, the inventors carried out the detection by means of an ion trap mass spectrometer working with electron impact ionization and a mass range between 50 and 600 amu in TIC Full Scan and SIM mode. The identification of compounds was carried out by matching with the retention time and mass spectrum of a glucose standard injected under the same conditions. This detection was carried out in the technical services of the Zaidín Experimental Station.

The inventors analyzed the characteristic functional groups of cellulose by means of attenuated total reflectance Fourier transform infrared spectroscopy (ATR/FTIR). Spectra were recorded with a JASCO FT/IR 6200 TypeA spectrophotometer equipped with the ATR PRO ONE attenuated total reflectance accessory and with SPECTRA MANAGER v2 software. They recorded the measurements after a mean of 100 scans with a resolution of 2 cm⁻¹ in the range of 400 to 4000 cm⁻¹. Before the analyses, freeze-dried samples were dried for 24 h in an oven at 60°C. These determinations were carried out with the equipment available at the Scientific Instrumentation Center of the University of Granada.

### Scanning electron microscopy (SEM)

The inventors performed specimen preparation and SEM analysis at the Scientific Instrumentation Center of the University of Granada. They carefully recovered the cellulose samples of the different cultures from the bottles after draining the medium, after which they were fixed for 2 h at 4°C in 2.5% glutaraldehyde prepared in cacodylate buffer at pH 7.4. Once fixed, the samples were washed three times (15 minutes each time) with the same buffer at 4°C and incubated for 1 h in 1 % osmium tetroxide at room temperature, after which they were washed three times for 5 min with water.

Subsequently, the inventors dehydrated the samples in an increasing gradient of ethanol from 50% to 100%. They dissected the samples further with carbon dioxide in a Leica EM CPD300 according to the protocol described in Anderson. et al. 1951, Trans NY Acad Sci 13, 130-134. The inventors covered the samples with carbon in an EMTECH K975X evaporator and observed them in a Zeiss SUPRA40VP scanning microscope equipped with a Schottky emission gun.

### RESULTS

### STN1B produces a biofilm growing with CO₂ as the sole carbon source

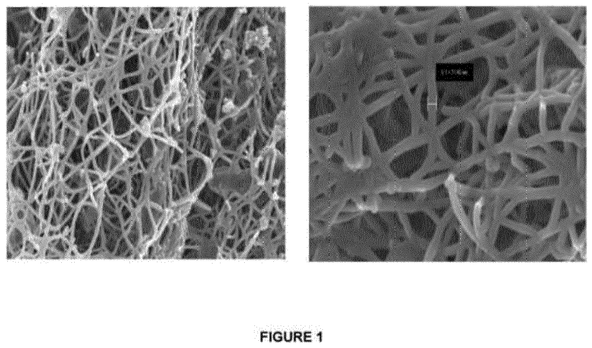

The inventors analyzed the capability of strain STN1B to produce cellulose from CO₂, in the form of sodium bicarbonate, using sodium thiosulfate as a reduced sulfur source. During its growth, the strain developed a spongy-looking biofilm at the bottom of the bottle, and its structure was analyzed using scanning electron microscopy, the results of which can be seen in Figure 1. The inventors observed that the strain *Starkeya* sp. STN1B produced extracellular nanocellulose with CO₂, a capability never previously described in other bacteria of the state of the art.

### Characteristics of the biofilm produced by STN1B

To visualize the structure of the biofilm matrix that forms when the strain is grown with bicarbonate as the sole carbon source, by means of scanning electron microscopy (SEM), the inventors demonstrated the presence of a three-dimensional lattice structure made of nanofibers about 70 nm in diameter and several microns in length (Figure 1). The network of fibers forms wide pores between the aforementioned fibers, and the presence of some bacilli about 1 µm in length, immersed in the network of nanofibers, in contact with one or more of these fibers, is observed, demonstrating that the strain produces an extracellular structure of bacterial nanocellulose.

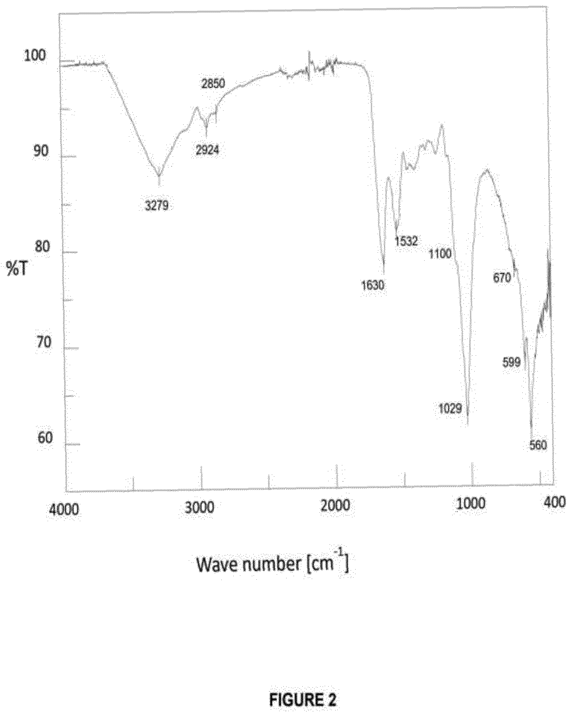

The inventors used attenuated total reflectance Fourier transform infrared (ATR-FTIR) spectroscopy to confirm the nature of the polymer. The results confirmed that the material obtained from the cultures with bicarbonate as the sole carbon source showed the characteristic profile of bacterial cellulose (Figure 2): a broad peak at 3279 cm⁻¹, corresponding to the stretching vibrations of the OH groups; peaks at 2914 and 2850 cm⁻¹, corresponding to the stretching vibrations of the CH groups; peaks at 1161 and 1105 cm⁻¹, corresponding to the vibration of the C-O-C bonds of the glycosidic bonds. This spectrum is compatible with type I cellulose, although the presence of some type II cellulose cannot be ruled out. Peaks at 1630 and 1532 cm⁻¹ are unexpected and could reflect the presence of water molecules or the presence of cell debris after treatment with KOH to eliminate the cells, which usually appear as peaks in the range of 1750-1400 cm⁻¹.

### STN1A produces a biofilm growing with CO₂ as the sole carbon source

Strain STN1A, a mutant strain with a deletion of a significant fragment of the STN1B genome in addition to a series of point mutations throughout its entire sequence, shows a significantly increased capability for the synthesis of cellulose. This strain growing in a container with a surface area of 160 cm² filled with 300 ml of PBT medium (i.e., with CO₂ and thiosulfate as the only carbon and energy sources, respectively) is capable of forming a consistent cellulose film.

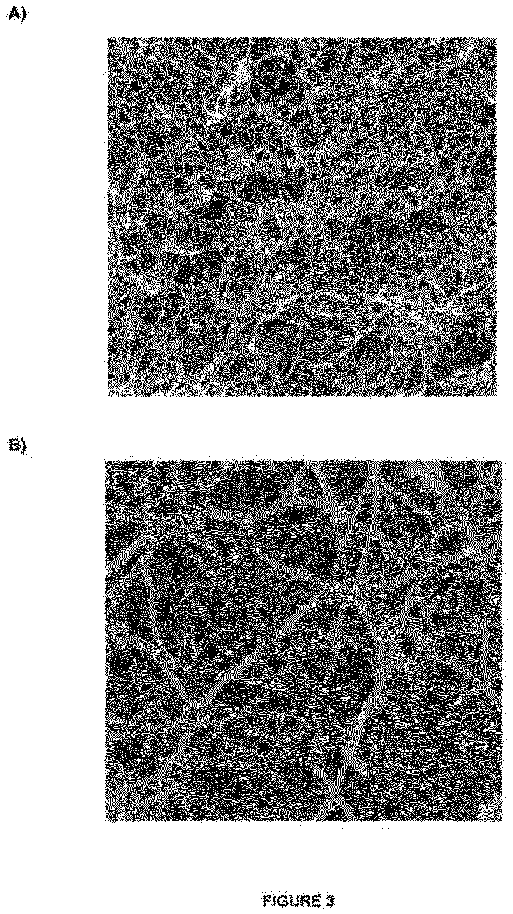

Like the biofilm formed by strain STN1B, the inventors demonstrated that the biofilm synthesized from CO₂ by strain STN1A had the typical characteristics of bacterial nanocellulose and was formed by fibers of several microns in length and between 60 and 70 nm in diameter (Figure 3A and 3B), leaving large gaps in the lattice. Within the lattice and in contact with the fibers, bacilli of about 1 µm in length were observed.

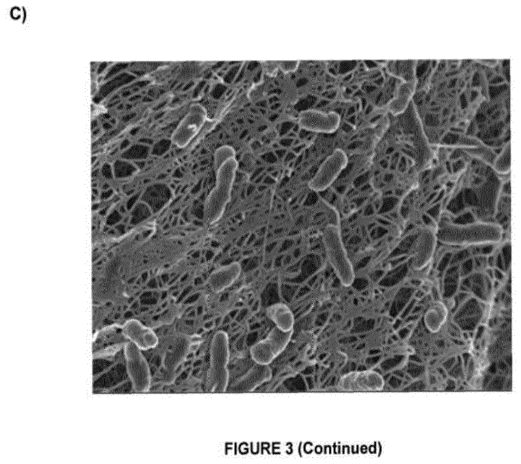

The structure formed by strain STN1A growing on glucose as a carbon source showed a similar structure, typical of bacterial nanocellulose (Figure 3C).

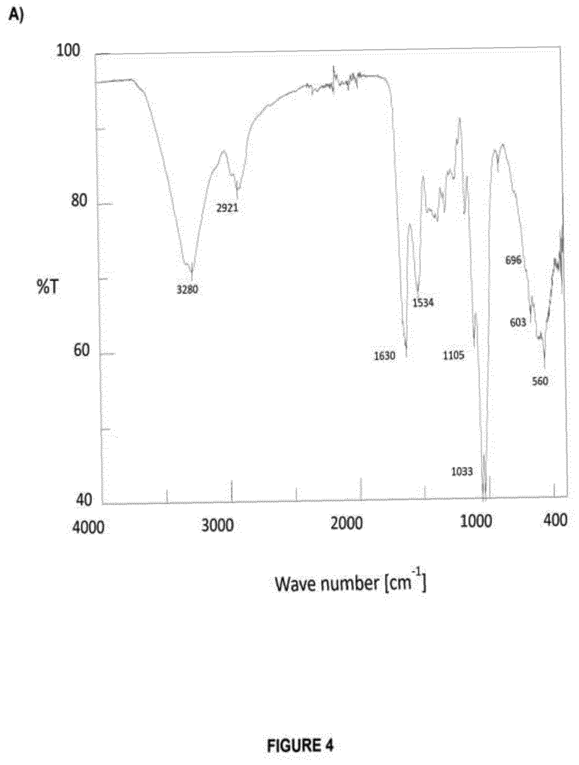

GC-MS chromatograms of the derivatized hydrolysis product of the aforementioned biofilm showed that glucose was the only component of the polymer. The inventors confirmed this by ATR-FTIR spectroscopy. Figure 4A shows a spectrum of the biofilm formed by STN1A growing with CO₂ as the sole carbon source, presenting a profile similar to that of strain STN1B, characteristic of bacterial cellulose.

### Strain STN1A presents a capability to produce cellulose with other carbon sources

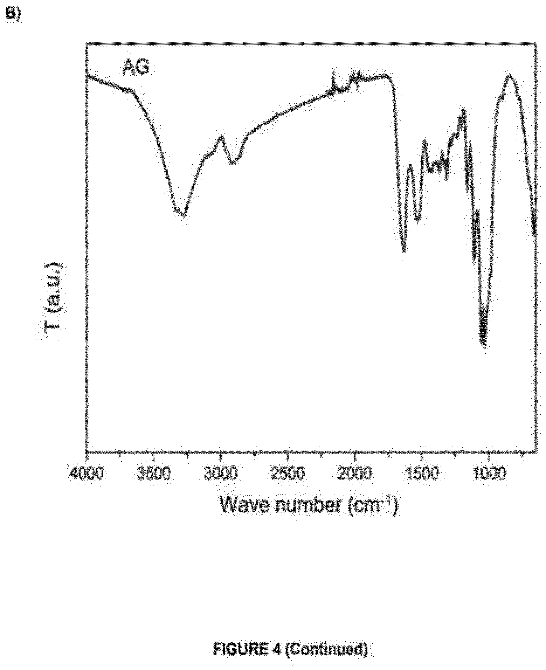

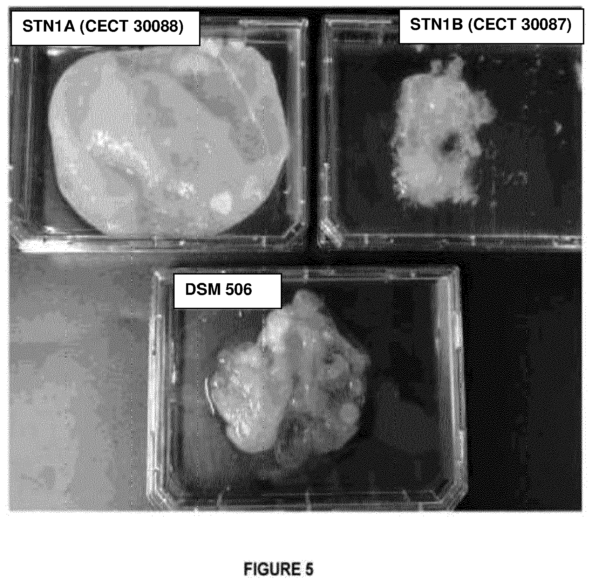

Using glucose or crude glycerin as a carbon source, strain STN1A is capable of synthesizing nanocellulose. The production of strain STN1B was 78.52 mg of nanocellulose per g of glucose consumed (106 mg of nanocellulose per 1.35 g of glucose), the production of strain DSM506 was 75.56 mg of nanocellulose per g of glucose consumed (170 mg of nanocellulose per 2.25 g of glucose), and the production of strain STN1A was much higher, 270.67 mg of nanocellulose per g of glucose consumed (609 mg of nanocellulose per 2.25 g of glucose) (Figure 5). Figure 4B shows the spectrum of the biofilm obtained from strain STN1A growing with glucose as the carbon source. The production can be compared with productions described in the state of the art, like the archetype *Komagataeibacter xylinus.* The production of nanocellulose by different strains of *Komagataeibacter xylinus* described in the literature and determined under the same drying conditions, ranges between 190 and 210 mg of nanocellulose per g of glucose consumed *(*Zhong C, et al., 2013. Appl Microbiol Biotechnol. 2013;97(14):6189-6199*;* Vazquez, A., et al., 2013. J Polym Environ, 21, 545-554), therefore, strain STN1A has higher production levels.

Furthermore, STN1A in medium with crude glycerin as the sole carbon source produces 289 mg of nanocellulose per g of glycerol consumed.

Furthermore, the crude glycerin can be UCO (used cooking oil) glycerin. STN1A in medium with crude UCO glycerin as the sole carbon source produces 150 mg of nanocellulose per g of UCO glycerin consumed.

### Biofilm is made up exclusively of cellulose

The chromatograms showed that the material extracted from the biofilms obtained was constituted exclusively of glucose, which constituted 98.2% of the carbohydrates present. The composition of the material extracted from a culture of the strain growing on glucose instead of bicarbonate as the sole carbon source was the same: exclusively glucose.

To confirm the cellulosic nature of the biofilm matrix, the inventors used mutants of strains STN1B and STN1A with a deletion in the gene *bcsA* (SEQ ID NO: 1) which encodes the major subunit of cellulose synthase, which generates an inactive truncated protein. They observed that inactivation of the gene *bcsA* was enough for the two mutant strains to lose the capability to form a biofilm when growing on bicarbonate as a carbon source, as well as when the source was glucose or glycerol.

### Type strain S. novella DSM 506 reproduces the capability for the synthesis of cellulose of STN1B

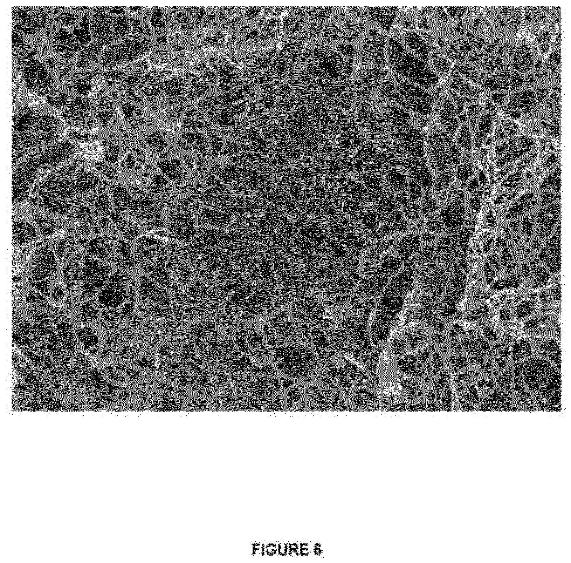

Cultures of strain DSM 506, both in PBT with CO₂ as the carbon source and in PBT medium with glucose or glycerol, were able to form a cellulose film similar to strain STN1B. Figure 6 shows the polymer network formed by DSM 506 growing with CO₂ as a carbon source in the presence of thiosulfate.

## Claims

1. A method for producing nanocellulose comprising:
(a) culturing at least one microorganism belonging to the genus *Starkeya,* or a population comprising a microorganism belonging to the genus *Starkeya,* in a culture medium comprising at least one carbon source, and
(b) purifying the nanocellulose biofilm obtained during step (a),
wherein the microorganism of the genus *Starkeya* is strain STN1A with deposit number CECT 30088.

2. The method according to claim 1, wherein the carbon source comprises CO₂, naphthalene, glycerol, glucose or any combination thereof.

3. The method according to claim 2, where the carbon source is CO₂.

4. The method according to claim 3, wherein the culture medium further comprises at least one reducing compound, preferably the reducing compound is a reduced sulfur compound and/or hydrogen gas.

5. The method according to any one of claims 1 to 4, wherein the culture of step (a) is carried out at a temperature between 16°C and 40°C, preferably between 20°C and 36°C, more preferably between 24°C and 32°C.

6. The method according to claim 5, wherein the temperature is 28°C.

7. The method according to any one of claims 1 to 6, wherein the culture medium is liquid.

8. The method according to any one of claims 1 to 7, wherein the culture is carried out under microaerophilic conditions and without stirring.

9. The method according to any one of claims 1 to 7, wherein the culture is carried out under stirring conditions between 25 and 150 rpm without air intake control, preferably between 50 and 100 rpm, more preferably at 75 rpm.

10. A nanocellulose polymer obtained by the method according to any one of claims 1 to 9.

11. A composition comprising the nanocellulose polymer according to claim 10.

12. Use of a microorganism belonging to the genus *Starkeya* or a population comprising a microorganism belonging to the genus *Starkeya* for the production of nanocellulose, wherein the production of nanocellulose is carried out in a culture medium comprising at least one carbon source, and wherein the microorganism of the genus *Starkeya* is strain STN1A with deposit number CECT 30088.

13. Use according to claim 12, wherein the carbon source is selected from the list consisting of: CO₂, naphthalene, glycerol, glucose and any combination thereof.

14. Use according to claim 12 or 13, wherein when the carbon source is CO₂, the culture medium further comprises at least one reducing compound, preferably the reducing compound is a reduced sulfur compound and/or hydrogen gas.

15. Use according to any one of claims 12 to 14, wherein the production of nanocellulose is carried out at a temperature between 16°C and 40°C, preferably between 20°C and 36°C, more preferably between 24°C and 32°C, still more preferably at 28°C.

16. Use according to any one of claims 12 to 15, wherein the culture medium is liquid.

17. Use according to any one of claims 12 to 16, wherein the production of nanocellulose is carried out under microaerophilic conditions and without stirring.

18. Use according to any one of claims 12 to 16, wherein the production of nanocellulose is carried out under stirring conditions between 25 and 150 rpm without air intake control, preferably between 50 and 100 rpm, more preferably at 75 rpm.

19. A strain of the genus *Starkeya* sp. (STN1A) with deposit number CECT 30088 or a strain derived therefrom, where the strain derived therefrom is a strain that results from a mutation or change in the DNA of one or more genes of strain CECT 30088 and that maintains the nanocellulose synthesis properties of strain CECT 30088.

20. A bacterial population comprising at least one strain of the genus *Starkeya* according to claim 19.

21. A composition comprising the strain according to claim 19 or the bacterial population according to claim 20.
